(19) Europäisches Patentamt
European Patent Office
Office européen des brevets

(11) Veröffentlichungsnummer: **0 218 970 B1**

(12) **EUROPÄISCHE PATENTSCHRIFT**

(45) Veröffentlichungstag der Patentschrift: **17.06.92**

(51) Int. Cl.[5]: **C07F 15/00**, B01J 31/24

(21) Anmeldenummer: **86113271.0**

(22) Anmeldetag: **26.09.86**

# (54) Chirale Rhodium-diphosphinkomplexe für asymmetrische Hydrierungen.

(30) Priorität: **18.10.85 CH 4498/85**

(43) Veröffentlichungstag der Anmeldung:
**22.04.87 Patentblatt 87/17**

(45) Bekanntmachung des Hinweises auf die Patenterteilung:
**17.06.92 Patentblatt 92/25**

(84) Benannte Vertragsstaaten:
**AT BE CH DE FR GB IT LI NL**

(56) Entgegenhaltungen:
**GB-A- 2 114 134**
**US-A- 3 660 493**
**US-A- 3 954 821**
**US-A- 4 343 741**

**JOURNAL OF ORGANOMETALLIC CHEMISTRY, Band 218, Nr. 2, September 1981, Seiten 249-260, Elsevier Sequoia S.A., Lausanne, CH; K. ACHIWA: "The mechanism of asymmetric hydrogenation catalysed by rhodium complexes of chiral pyrrolidinobiphosphines"**

**JOURNAL OF ORGANOMETALLIC CHEMISTRY, Band 288, Nr. 2, 18. Juni 1985, Seiten C37-C39, Elsevier Sequoia S.A., Lausanne, CH; R. BENHAMZA et al.: "Synthesis and application of rhodium complexes of asymmetric water-soluble diphosphine derived from 2-[(diphenylphosphino)methyl]-4-(diphenylphosphino)-pyrrolidine"**

(73) Patentinhaber: **F. HOFFMANN-LA ROCHE AG**
**Postfach 3255**
**CH-4002 Basel(CH)**

(72) Erfinder: **Broger, Emil Albin, Dr.**
**Obere Egg 11**
**CH-4312 Magden(CH)**
Erfinder: **Crameri, Yvo, Dr.**
**Hohestrasse 103**
**CH-4104 Oberwil(CH)**

(74) Vertreter: **Cottong, Norbert A. et al**
**Grenzacherstrasse 124 Postfach 3255**
**CH-4002 Basel(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die vorliegende Erfindung betrifft neue chirale Rhodium-diphosphinkomplexe der allgemeinen Formel

$$[Rh(X)(Y)(L_{0,1 \text{ oder } 2})]_{1 \text{ oder } 2} \qquad I$$

worin X, welches gegebenenfalls auf einem Träger fixiert sein kann, einen Rest der Formel Z-$COO^-$ darstellt, worin Z wiederum eine Gruppe

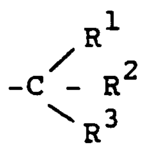
,
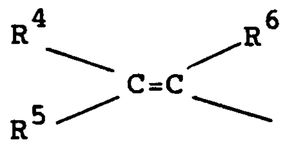

oder Aryl bedeutet, worin

$R^1$, $R^2$, $R^3$ = Wasserstoff. Halogen, niederes Alkyl, Aryl-niederes Alkyl, Perfluor-$C_{1-20}$-Alkyl, Aryl oder die Gruppe -$OR^7$, -$(CH_2)_n$-COA oder AOC-$(CF_2)$n bedeuten, wobei wenigstens einer von $R^1$, $R^2$ und $R^3$ -$OR^7$ oder Aryl darstellt,
$R^4$, $R^5$, $R^6$ = Wasserstoff, Halogen, niederes Alkyl, Aryl-niederes Alkyl, Perfluor-$C_{1-20}$-Alkyl, Aryl oder die Gruppe -$(CH_2)_n$-COA oder AOC-$(CF_2)_n$ bedeuten
$R^7$ = Wasserstoff, niederes Alkyl, teilweise oder vollständig halogeniertes niederes Alkyl, Aryl oder Aryl-niederes Alkyl darstellt
A = die Reste -OR oder -$NR'_2$ bedeutet
R = Wasserstoff, niederes Alkyl, Aryl, Aryl-niederes Alkyl oder ein Kation bedeutet,
R' = Wasserstoff, niederes Alkyl, Aryl oder Aryl-niederes Alkyl bedeutet,
n = eine Zahl 0 bis 20 bedeutet, ferner Y einen chiralen Diphosphinliganden und L einen neutralen Liganden darstellen,

mit Ausnahme derjenigen Komplexe der Formel I, worin X, welches gegebenenfalls auf einem Träger fixiert sein kann, einen Rest der Formel Z-$COO^-$ darstellt, worin Z wiederum die Gruppe

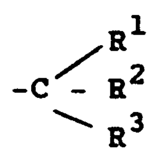

Perfluorphenyl, Perfluorbiphenyl oder einen Rest der Formel

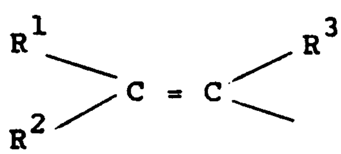

bedeutet und $R^1$, $R^2$ und $R^3$ Halogen, niederes Alkyl, Perfluorphenyl, Perfluor-$C_{1-20}$-Alkyl, Wasserstoff oder die Gruppe -COA oder AOC-$(CF_2)_n$- darstellen, worin A die Reste -OR oder -$NR'_2$ bedeutet, wobei jedoch wenigstens einer der Substituenten $R^1$, $R^2$ und $R^3$ Fluor bedeutet, R Wasserstoff, niederes Alkyl oder ein Kation, R' Wasserstoff oder niederes Alkyl, und n eine Zahl 1 bis 20 bedeuten, wobei die niederen Alkylreste 1-9 Kohlenstoffatome aufweisen und der Ausdruck Aryl für gegebenenfalls substituierte aromatische Kohlenwasserstoffe oder aromatische Heterocyclen mit 4-14 Kohlenstoffatomen steht.

Die Erfindung betrifft ferner die Herstellung der Rhodium-diphosphinkomplexe der Formel I, sowie deren Verwendung für asymmetrische Hydrierungen.

Aus der Literatur sind bereits chirale Rhodium-diphosphinkomplexe und deren Verwendung bei asymmetrischen Hydrierungen bekannt. Ueblicherweise sind diese Komplexe kationisch oder enthalten - falls

neutral - als Ligand X Chlor, Brom oder Jod. Die optischen Ausbeuten welche bei der Verwendung derartiger Komplexe in asymmetrischen Hydrierungen erzielt werden, liegen in den günstigsten Fällen bei etwa 80-84%, im Falle der Hydrierung von Ketopantolacton [J. Org. Chem., Vol.43, No 18 (1978) 3444-3446].

Aus J. Organometallic Chem., Bd. 218, No2, Sept. 1981, Seiten 249-260 und Bd. 288, No2, Juni 1985, Seiten C 37-C 39 sind weiterhin kationische Rhodiumkomplexe bekannt, mit einem chiralen PPM-Liganden, bzw. einem davon durch Substitution am H-Atom des Pyrrolidins abgeleiteten Liganden, wie beispielsweise BPPM, gegebenenfalls einem weiteren, neutralen Liganden und, im Unterschied zur vorliegenden Anmeldung, mit $BF_4^{\ominus}$ beziehungsweise $ClO_4^{\ominus}$ als Gegenion.

Ausserdem sind in EP-A-0158875 den in vorliegender Anmeldung beanspruchten Rhodium-diphosphinkomplexen sehr ähnliche Komplexe beschrieben, wobei es sich bei dieser Literaturstelle jedoch lediglich um einen Stand der Technik nach Art. 54(3) EPUe handelt, welchem bereits entsprechend Rechnung getragen wurde.

Ueberraschenderweise wurde nun gefunden, dass die erfindungsgemässen Rhodium-diphosphinkomplexe der Formel I im Vergleich zu den vorbekannten erheblich aktiver und enantioselektiver sind, was insbesondere dazu führt, dass durch ihre Verwendung erheblich geringere Katalysatormengen verwendet werden können, kürzere Reaktionszeiten möglich sind und optische Ausbeuten von über 95% erzielt werden können.

Der Ausdruck "niederes Alkyl" bedeutet im Rahmen der vorliegenden Erfindung geradkettige oder verzweigte Alkylgruppen mit 1 bis 9 Kohlenstoffatomen wie z.B. Methyl, Aethyl, Propyl, Isopropyl, n-Butyl, Isobutyl, tert. Butyl, Pentyl, Hexyl, Heptyl, Octyl oder Nonyl und dergleichen. Der Ausdruck "Halogen" bedeutet Fluor, Chlor, Brom und Jod, wobei Fluor bevorzugt ist. Der Ausdruck "Perfluor-$C_{1-20}$-Alkyl" bedeutet im Rahmen der vorliegenden Erfindung sowohl geradkettige, als auch verzweigte, gegebenenfalls auch optisch aktive Ketten, wobei nicht unbedingt sämtliche Wasserstoffatome durch Fluoratome ersetzt sein müssen. Falls nicht alle Wasserstoffatome durch Fluoratome ersetzt sind, so ist häufig insbesondere noch ein endständiges Wasserstoffatom vorhanden. Sofern X auf einem Träger fixiert ist, erfolgt dies über eine Gruppe -COA.

Der im Zusammenhang mit den Verbindungen der Formel I verwendete Ausdruck "Aryl" bedeutet, im Rahmen der vorliegenden Erfindung, sowohl aromatische Kohlenwasserstoffe als auch aromatische Heterocyclen mit 4 bis 14 Kohlenstoffatomen. Als Heteroatome kommen insbesondere in Betracht Sauerstoff und Stickstoff. Weiterhin können die Ringe sowohl unsubstituiert als auch substituiert sein, wobei als Substituenten bevorzugt in Betracht kommen, Halogen, Hydroxy, niederes Alkyl, Perfluor-niederes Alkyl, niederes Alkoxy und Formyl. Eine vorhandene Arylgruppe kann zudem an ein Uebergangsmetall wie Chrom, Eisen oder auch Nickel komplexiert sein.

Der im nachfolgenden, im Zusammenhang mit den Verbindungen der Formel II verwendete Ausdruck "Aryl" bedeutet im Rahmen der vorliegenden Erfindung Phenyl, welches gegebenenfalls in para- und/oder meta-Stellung niedere Alkyl- oder niedere Alkoxygruppen, vorzugsweise Methyl- oder Methoxygruppen, oder auch di-niederes Alkylamino, vorzugsweise Dimethylaminogruppen, sowie eine Carboxy-, Carbamoyl-, Cyano- oder eine niedere Alkoxycarbonylgruppe aufweisen kann. Zudem können zwei Arylgruppen am gleichen Phosphoratom über die o-Stellung direkt miteinander verbunden sein oder auch über eine Methylen-, Aethylen- oder Propylengruppe. Der Ausdruck "Aryloxy" bedeutet Gruppen, in denen der Arylrest die vorhergehende Bedeutung hat.

Der Ausdruck "niederes Alkoxy" bedeutet Gruppen, in denen der Alkylrest die vorhergehende Bedeutung hat. Weiterhin bedeutet das Zeichen "◄", dass sich der entsprechende Rest oberhalb der Molekülebene befindet, während das Zeichen " " bedeutet, dass sich der entsprechende Rest unterhalb der Molekülebene befindet. Der Buchstabe n bedeutet eine Zahl von 0 bis 20, vorzugsweise von 1 bis 12, insbesondere von 1 bis 8.

Der Ausdruck "neutraler Ligand" bedeutet im Rahmen der vorliegenden Erfindung einen leicht austauschbaren Liganden wie Olefine, z.B. Aethylen, Propylen, Cycloocten, 1,5-Hexadien, Norbornadien, 1,5-Cyclooctadien und dergleichen, Nitrile wie Acetonitril, Benzonitril, oder auch das verwendete Lösungsmittel usw. Dieser Ligand kann bei der Hydrierung ausgetauscht werden. Falls mehr als ein solcher Ligand vorhanden ist, können diese auch voneinander verschieden sein.

Als chirale Diphosphinliganden können im Prinzip beliebige, im Zusammenhang mit asymmetrischen Hydrierungen bekannte Diphosphinliganden, welche gegebenenfalls auch auf einem Träger fixiert sein können, verwendet werden. Der artige Liganden sind bekannt und dem Fachmann leicht zugänglich. Beispielsweise sind im Rahmen der vorliegenden Erfindung in Frage kommende Liganden bekannt aus: Marko, L. et al., Aspects of Homogenous Catalysis, 4, 145-202 (1981); Japanische Patentanmeldung No. 67411 vom 4.6.1978 (Derwent 8180 C); deutsche Offenlegungsschrift No. 2 161 200; Europäische Patentpublikation No. 104 375. Besonders geeignete und bevorzugte Liganden sind z.B. die chiralen Phosphine der allgemeinen Formel

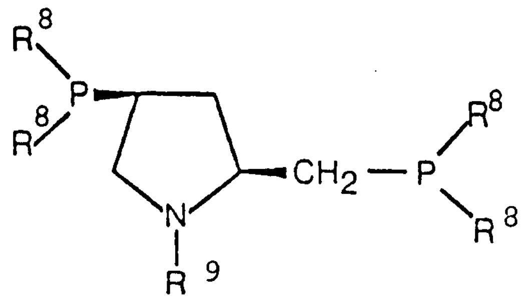

II

worin $R^8$ Aryl und $R^9$ die Gruppen $-CO-R^{10}$, $-SO_2-R^{10}$, $-PO(R^{10})_2$ oder $-PS(R^{10})_2$ darstellen, wobei $R^{10}$ Aryl, niederes Alkyl, di-Arylamino, di-niederes Alkylamino, Aryloxy oder niederes Alkoxy bedeuten, wobei die niederen Alkyl- bzw. Alkoxyreste 1-9 Kohlenstoffatome aufweisen und der Ausdruck Aryl für sich oder in Aryloxy für gegebenenfalls substituiertes Phenyl steht.

Bevorzugte Rhodium-diphosphinkomplexe der Formel I sind solche, worin Z die Gruppe

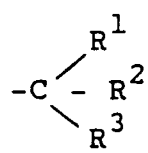

darstellt und einer der Substituenten $R^1$, $R^2$ und $R^3$ den Rest $-OR^7$ und die beiden anderen Fluor, Wasserstoff, Perfluor-$C_{1-20}$-Alkyl oder Aryl bedeuten, sowie die jenigen, worin einer der Substituenten $R^1$, $R^2$ und $R^3$ Aryl und die beiden anderen Fluor, Wasserstoff oder Perfluor-$C_{1-20}$-Alkyl darstellen, wobei jedoch wenigstens einer davon Fluor bedeutet. Falls der Ligand X chiral ist, kann dieser in racemischer oder bevorzugt in optisch aktiver Form vorliegen.

Bevorzugte Diphosphinliganden der Formel II sind solche, worin $R^8$ Phenyl, p-Tolyl, m-Tolyl oder 3,5-Xylyl darstellt und $R^{10}$ in den Resten $R^9$ Phenyl, p-Tolyl, m-Tolyl, p-nied. Alkoxycarbonylphenyl oder tert. Butoxy bedeutet. Besonders bevorzugte Phosphine sind zudem die jenigen, worin der Rest $R^9$ die Gruppe $-PO(R^{10})_2$ darstellt. Als Beispiele von bevorzugten Diphosphinliganden können folgende genannt werden:

(2S,4S)-4-(Di-m-Tolylphosphino)-2-[(di-m-tolylphosphino) methyl]-1-(diphenylphosphinoyl)-pyrrolidin; ($mCH_3$-POPPM)

(2S,4S)-4-(Diphenylphosphino)-2-[(diphenylphosphino) methyl]-1-(diphenylphosphinoyl)-pyrrolidin: (POPPM)

(2S,4S)-4-(Di-p-Tolylphosphino)-2-[(di-p-tolylphosphino) methyl]-1-(diphenylphosphinoyl)-pyrrolidin; ($pCH_3$-POPPM)

(2S,4S)-4-(Diphenylphosphino)-2-[(diphenylphosphino) methyl]-1-(di-p-carbomethoxyphenylphosphinoyl)-pyrrolidin;

(2S,4S)-4-(Diphenylphosphino)-2-[(diphenylphosphino) methyl]-1-(tert. butoxycarbonyl)-pyrrolidin; (BPPM)

(2S,4S)-4-(Di-m-Tolylphosphino)-2-[(di-m-tolylphosphino) methyl]-1-(tert. butoxycarbonyl)-pyrrolidin: ($mCH_3$-BPPM)

Besonders bevorzugte Rhodium-diphosphinkomplexe der Formel I sind diejenigen, worin einer der Substituenten $R^1$, $R^2$ und $R^3$ den Rest $-OR^7$ und die beiden anderen Fluor oder einer Trifluormethyl und der andere Phenyl bedeuten, sowie diejenigen, worin einer der Substituenten $R^1$, $R^2$ und $R^3$ Phenyl und die beiden anderen Fluor oder einer Wasserstoff und der andere Fluor darstellen und der Diphosphinligand $mCH_3$-POPPM ist. Ganz besonders bevorzugt sind die Komplexe der Formeln:

$[Rh(C_6F_5-O-CF_2-COO)(mcH_3-POPPM)(L_{0,1\ oder\ 2})]_{1\ oder\ 2}$
$[Rh((R)-C_6H_5-C(CF_3)(OCH_3)-COO)(mCH_3-POPPM)(L_{0,1\ oder\ 2})]_{1\ oder\ 2}$
$[Rh(C_6H_5-CHF-COO)(mCH_3-POPPM)(L_{0,1\ oder\ 2})]_{1\ oder\ 2}$

Die erfindungsgmässen Rhodium-diphosphinkomplexe der Formel I können in an sich bekannter Weise hergestellt werden. Sie können beispielsweise dadurch hergestellt werden. dass man

a) einen Rhodiumkomplex der Formel

$[Rh\ (X)\ (L_m)]_{1\ oder\ 2}$  III

worin X und L obige Bedeutung haben, und m eine Zahl von 1 bis 4 bedeutet,
mit einem chiralen Diphosphinliganden umsetzt, oder

b) einen Rhodiumkomplex der Formel

$[Rh\ (L_{m+1})]^+\ A^-$  IV

worin L und m obige Bedeutung haben und $A^-$ ein Anion, insbesondere $BF_4^-$, $ClO_4^-$, $PF_6^-$ oder $B(C_6H_5)_4^-$ darstellt,
mit einem chiralen Diphosphinliganden und einem das Anion X enthaltenden Salz umsetzt, oder

c) einen Rhodium-diphosphinkomplex der Formel

$[Rh\ (L_p)\ (Y)]^+\ A^-$  V

worin L, Y und $A^-$ obige Bedeutung haben und p eine Zahl von 1 bis 3 darstellt,
mit einem das Anion X enthaltenden Salz umsetzt, oder

d) einen chiralen Rhodium-diphosphinkomplex der Formel

$[Rh\ (X^1)\ (L_{1\ oder\ 2})\ (Y)]$  VI

worin $X^1$ Halogen bedeutet und L und Y obige Bedeutung haben,
mit einem Silbersalz oder Thalliumsalz der Formel

Ag - X oder Tl - X  VII

worin X obige Bedeutung hat,
umsetzt.

Die Umsetzungen der Rhodiumkomplexe der Formeln III, IV, V und VI gemäss den Reaktionsvarianten a) - d) können in an sich bekannter Weise durchgeführt werden. Zweckmässig erfolgt dies in einem inerten organischen Lösungsmittel. Als Beispiele hierfür können genannt werden: aromatische Kohlenwasserstoffe wie Benzol, Toluol usw., Ester wie Essigester, usw., cyclische Aether wie Tetrahydrofuran oder Dioxan, niedere Alkohole wie Methanol, Aethanol und dergleichen oder auch Gemische hiervon. Die Umsetzung kann bei Temperaturen zwischen etwa 0°C bis etwa 100°C, vorzugsweise bei etwa 15°C bis etwa 60°C erfolgen, jedoch unter striktem Ausschluss von Sauerstoff.

Der Ausdruck "das Anion X enthaltende Salz" bedeutet im Rahmen der vorliegenden Erfindung insbesondere Ammoniumsalze, Alkalimetallsalze, Erdalkalimetallsalze sowie andere geeignete Metallsalze. Derartige Salze sind bekannte Substanzen. Um die Löslichkeit derartiger Salze in gewissen Lösungsmitteln zu erhöhen kann gegebenenfalls ein geeigneter Kronenäther zugesetzt werden.

Bei den Rhodium-diphosphinkomplexen der Formel I handelt es sich um Katalysatoren bzw. um Vorstufen hiervon. Da deren genaue chemische Struktur nicht mit Sicherheit angegeben werden kann, sind sie auch dadurch gekennzeichnet, dass sie erhältlich sind, durch Umsetzung eines Rhodiumkomplexes der Formeln III-VI gemäss den vorhergehend erwähnten Reaktionen a) bis d).

Die als Ausgangsmaterialien verwendeten Rhodiumkomplexe der Formeln III, IV, V und VI sind bekannte Substanzen oder Analoge bekannter Substanzen, welche leicht in Analogie zu den bekannten hergestellt werden können.

Wie bereits erwähnt dienen die erfindungsgemässen Rhodium-diphosphinkomplexe der Formel I als Katalysatoren bei asymmetrischen Hydrierungen. Besonders interessant sind sie im Zusammenhang mit der asymmetrischen Hydrierung von $\alpha,\beta$-ungesättigten Säuren und Estern sowie von $\alpha$-Keto-carbonsäuren und Estern und von $\alpha$-Keto-Lactonen. Insbesondere sind sie von Interesse zur asymmetrischen Hydrierung von Dihydro-4,4-dimethyl-2,3-furandion (Ketopantolacton) zum entsprechenden R-($\alpha$-Hydroxy-$\beta,\beta$-dimethyl-$\gamma$-butyrolacton)[R-(-)-Pantolacton].

Zur Durchführung der erwähnten asymmetrischen Hydrierungen können die Komplexe der Formel I als solche zu einer Lösung einer asymmetrisch zu hydrierenden Verbindung gegeben werden. Andererseits

können sie auch in situ in Gegenwart einer asymmetrisch zu hydrierenden Verbindung gebildet werden.

Die asymmetrischen Hydrierungen können in geeigneten, unter den Reaktionsbedingungen inerten organischen Lösungsmitteln durchgeführt werden. Als solche können insbesondere genannt werden niedere Alkanole wie z.B. Methanol oder Aethanol, aromatische Kohlenwasserstoffe wie Benzol oder Toluol, cyclische Aether wie Tetrahydrofuran oder Dioxan, Ester wie z.B. Essigester oder auch Gemische hiervon und dergleichen. Das Verhältnis zwischen Rhodium und den Liganden Y liegt zweckmässig zwischen etwa 0,05 und etwa 5 Mol, vorzugsweise zwischen etwa 0,5 und etwa 2 Mol Rhodium pro Mol Ligand. Das Verhälnis zwischen Rhodium und dem Rest X liegt zweckmässig zwischen etwa 0,01 und etwa 20, vorzugsweise zwischen etwa 0,5 und etwa 10 Mol Rhodium pro Mol Rest X. Das Verhältnis zwischen Rhodium, in den Komplexen der Formel I, und den zu hydrierenden Verbindungen liegt zweckmässig zwischen etwa 0,00001 und etwa 5 Gew.%, vorzugsweise zwischen etwa 0,0001 und etwa 0,01 Gew.%.

Die asymmetrischen Hydrierungen unter Verwendung der Komplexe der Formel I können zweckmässig bei Temperaturen von etwa 0 ° C bis etwa 100 ° C, vorzugsweise von etwa 20 ° C bis etwa 70 ° C, durchgeführt werden. Diese Hydrierungen erfolgen zweckmässig unter Druck, insbesondere unter einem Druck von etwa 1 bis 100 bar, vorzugsweise 2 bis 50 bar.

Die folgenden Beispiele dienen zur Erläuterung der Erfindung und stellen keinerlei Einschränkung hiervon dar.

In diesen Beispielen haben die verwendeten Abkürzungen folgende Bedeutung:

COD = 1,5-Cyclooctadien

BPPM; m$CH_3$-POPPM: auf Seite 6 erwähnte Diphosphine.

Die enantiomere Zusammensetzung (Enantiomerenüberschuss e.e.) wurde durch gaschromatographische Bestimmung von (R)- und (S)-Pantolacton auf einer 25m Kapillarsäule mit chiraler Phase (SP-300) bestimmt. In mehreren Fällen wurden zusätzlich die optischen Drehungen von (R)-(-)-Pantolacton bei 589 nm (D-Linie) bei 20 ° C und einer Konzentration von 3% in ionenfreiem Wasser gemessen. Die Werte für die optischen Reinheiten basieren auf $[\alpha]_D^{20}$ = -51,6 ° (c = 3, $H_2O$) für reinstes (R)-(-)-Pantolacton.

Beispiel 1

In einer Glove-Box ($O_2$-Gehalt <1 ppm) wurden in einem 100 ml Glaskolben 254,2 mg (0,389 mMol) einer 40%igen wässerigen Tetrabutylammoniumhydroxid-Lösung, 108,2 mg (0,389 mMol) Perfluorphenoxyessigsäure, 157,8 mg (0,389 mMol) Bis-(1,5-Cyclooctadien)-rhodium(I)tetrafluoroborat und 281,7 mg (0,389 mMol) (2S,4S)-4-(Di-m-Tolylphosphino)-2-[(di-m -tolylphosphino)methyl]-1-(diphenylphosphinoyl)-pyrrolidin (m$CH_3$-POPPM) in 50 ml Toluol suspendiert. Die Suspension wurde anschliessend während 16 Stunden bei 22-25 ° C gerührt, wobei sich eine orange-rote, nahezu klare Katalysatorlösung bildete.

Beispiel 2

In einer Glove-Box ($O_2$-Gehalt <1 ppm) wurde ein 500 ml-Autoklav mit 40 g (0,31 Mol) Ketopantolacton, 160 ml Toluol und 50 ml nach Beispiel 1 hergestellter Katalysatorlösung beladen. Die Hydrierung wurde bei 40 ° C, einem konstanten Druck von 40 bar $H_2$ und unter intensivem Rühren durchgeführt. Nach 1 Stunde betrug der Umsatz 99.8%. Nach total 4 Stunden Hydrierzeit wurde die gelbe Hydrierlösung aus dem Autoklaven gespült und der Autoklav anschliessend dreimal mit je 50 ml Toluol nachgespült. Die vereinigten Toluollösungen wurden am Rotationsverdampfer bei 60 ° C/17 mbar eingedampft. Der Rückstand (42 g) wurde bei 130-150 ° C Badtemperatur und 12 mbar destilliert. Man erhielt 40,3 g (99,3%) (R)-α-Hydroxy-β,β-dimethyl-γ-butyrolacton (= (R)-(-)-Pantolacton), mit einer optischen Reinheit von 92,8% e.e.

Beispiele 3-29

In zu Beispiel 1 analoger Weise wurde eine Katalysatorlösung hergestellt und anschliessend die Hydrierung von Ketopantolacton analog Beispiel 2 durchgeführt. Die Resultate sind in der nachfolgenden Tabelle zusammengefasst:

| Beispiel Nr. | [Rh (X)(Y)(L)] 1) X | Y | Solvens | Druck [bar] | % Umsatz nach 1 Stunde | (R)-(-)-Pantolacton e.e. % | Bem. |
|---|---|---|---|---|---|---|---|
| 3 | 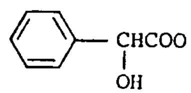 [S] | m-$CH_3$POPPM | Toluol | 40 | 99,3 | 90,6 | |
| 4 | 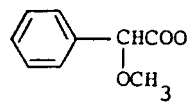 [R] | m-$CH_3$POPPM | " | 40 | 99,6 | 91,2 | 2) |
| 5 | 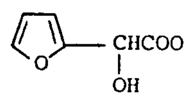 [R,S] | m-$CH_3$POPPM | " | 40 | 99,4 | 87,9 | |
| 6 a | 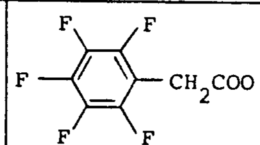 | m-$CH_3$POPPM | " | 40 | 99,8 | 91,8 | |
| 6 b | | BPPM | " | 40 | 95,2 | 89,2 | |
| 7 | 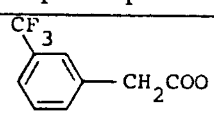 | m-$CH_3$POPPM | " | 40 | 96,1 | 90,4 | |
| 8 | 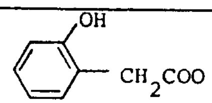 | m-$CH_3$POPPM | " | 40 | 99,9 | 92,0 | |
| 9 | 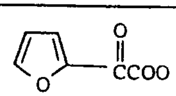 | m-$CH_3$POPPM | " | 40 | 99,6 | 89,7 | |

| Beispiel Nr. | [Rh (X)(Y)(L)] 1) X | | Y | Solvens | Druck [bar] | % Umsatz nach 1 Stunde | (R)-(-)-Pantolacton e.e. % | Bem. |
|---|---|---|---|---|---|---|---|---|
| 10 | 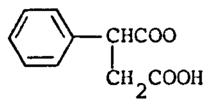 | [R,S] | m-$CH_3$POPPM | Toluol | 40 | 92,5 | 90,0 | 3) |
| 11 a | 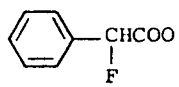 | [R,S] | m-$CH_3$POPPM | " | 40 | 99,7 | 94,6 | |
| 11 b | | | BPPM | " | 40 | 98,4 | 88,5 | |
| 12 | 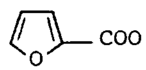 | | m-$CH_3$POPPM | " | 40 | 99,7 | 90,0 | |
| 13 | 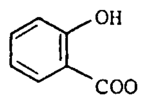 | | m-$CH_3$POPPM | " | 40 | 59,5 | 89,9 | 4) |
| 14 | 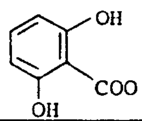 | | m-$CH_3$POPPM | " | 40 | 99,6 | 92,4 | |
| 15 | 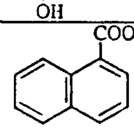 | | m-$CH_3$POPPM | " | 40 | 96,2 | 87,9 | |
| 16 | 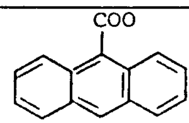 | | m-$CH_3$POPPM | " | 40 | 99,5 | 91,2 | |
| 17 | 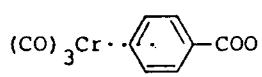 | | m-$CH_3$POPPM | " | 40 | 99,6 | 90,0 | |

| Beispiel Nr. | [Rh (X)(Y)(L)] 1) X | Y | Solvens | Druck [bar] | % Umsatz nach 1 Stunde | (R)-(-)-Pantolacton e.e. % | Bem. |
|---|---|---|---|---|---|---|---|
| 18 | 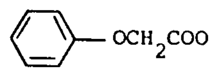 | m-$CH_3$POPPM | Toluol | 12 | 99,9 | 94,8 | |
| 19 | 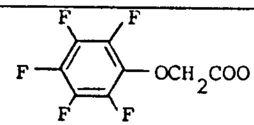 | m-$CH_3$POPPM | " | 12<br>40 | 98,4<br>99,7 | 92,3<br>92,5 | |
| 20 | 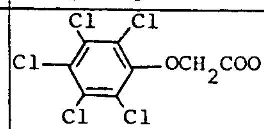 | m-$CH_3$POPPM | " | 12<br>40 | 99,4<br>99,9 | 94,1<br>94,8 | |
| 21 | $CH_3OCH_2COO$ | m-$CH_3$POPPM | " | 40 | 99,2 | 92,5 | 5) |
| 22 | $C_2H_5OCH_2COO$ | m-$CH_3$POPPM | " | 40 | 99,1 | 90,0 | |
| 23 a<br>b<br>c<br>d | 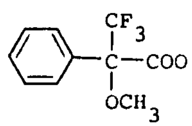 [ R ] | m-$CH_3$POPPM<br>m-$CH_3$POPPM<br>m-$CH_3$POPPM<br>BPPM | "<br>Essigester<br>THF<br>Toluol | 12<br>12<br>12<br>40 | 99,1<br>77,1<br>73,1<br>56,5 | 95,6<br>94,8<br>94,0<br>92,2 | 6)<br><br><br>7) |
| 24 | [ S ] | m-$CH_3$POPPM | Toluol | 12 | 98,2 | 92,5 | |
| 25 | 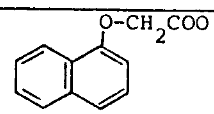 | m-$CH_3$POPPM | " | 40 | 100,0 | 89,9 | |

| Beispiel Nr. | [Rh (X)(Y)(L)] 1) X | Y | Solvens | Druck [bar] | % Umsatz nach 1 Stunde | (R)-(-)-Pantolacton e.e. % | Bem. |
|---|---|---|---|---|---|---|---|
| 26 | 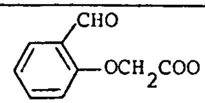 | m-$CH_3$POPPM | Toluol | 40 | 98,7 | 93,0 | |
| 27 | 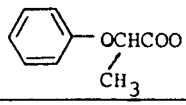 [R,S] | m-$CH_3$POPPM | " | 40 | 99,6 | 90,7 | |
| 28 | $(CH_3O)_2CHCOO$ | m-$CH_3$POPPM | " | 40 | 97,8 | 90,5 | |
| 29 a | 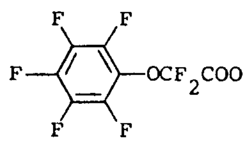 | m-$CH_3$POPPM | " | 12 | 68,5 | 92,6 | 8) |
| 29 b | | BPPM | " | 40 | 99,9 | 92,5 | |

1) L = 1,5-Cyclooctadien

2) $[\alpha]_D^{20}$ = -47,2 ° (c=3, $H_2O$), opt. Reinheit 91,4%

3) $[\alpha]_D^{20}$ = -46,24° (c=3, $H_2O$), opt. Reinheit 89,6%

4) $[\alpha]_D^{20}$ = -45,93° (c=3, $H_2O$), opt. Reinheit 89,0%

5) $[\alpha]_D^{20}$ = -47,8° (c=3, $H_2O$), opt. Reinheit 92,6%

6) $[\alpha]_D^{20}$ = -48,7° (c=3, $H_2O$), opt. Reinheit 94,4%

7) S/Rh = 15 720 (Gewichtsverhältnis Ketopantolacton zu Rhodiummetall)

8) S/Rh = 62 880 ( " " " )

Beispiel 30

In einer Glove-Box ($O_2$-Gehalt <1 ppm) wurde ein 500 ml-Stahlautoklav beladen mit 30 g (0,104 Mol) α-Oxo-γ-(1,3-dioxo-2-isoindolyl) -buttersäure-isopropylester, 110 ml Toluol und einer analog Beispiel 1, aus 165 mg (0,292 mMol) einer 40%igen Tetrabutylammoniumhydroxid-Lösung, 68,3 mg (0,292 mMol) (S)-α-Methoxy-α-trifluormethylphenylessigsäure, 118,4 mg (0,292 mMol) Bis-(1,5-Cyclooctadien)-rhodium(I)-tetrafluoroborat und 164,7 mg (0,292 mMol) (2S,4S)-1-tert-Butoxycarbonyl-4-diphenylphosphino -2-diphenylphosphinomethyl-pyrrolidin hergestellter Katalysatorlösung. Die Hydrierung erfolgte bei einem konstanten Druck von 10 bar $H_2$, bei 40°C und unter intensivem Rühren während 24 Stunden. Die Hydrierlösung wurde eingedampft, der Rückstand an Kieselgel chromatographiert und die das Produkt enthaltenden Fraktionen eingedampft. Man erhielt 29,5 g (97,4%) (R)-α-Hydroxy-γ-(1,3-dioxo -2-isoindolyl)-buttersäureisopropylester als hellgelbe Kristalle. Smp. 78-82°C. Enantiomere Reinheit 78,5% (gemäss GC-Bestimmung auf einer 21m Kapillarsäule [Se 54] als Camphansäureester). $[\alpha]_D^{20}$ = -4,4° (c=1, $CH_3OH$); optische Reinheit 79,0%.

## Patentansprüche

**Patentansprüche für folgende Vertragsstaaten : BE, CH, DE, FR, GB, IT, LI, NL**

**1.** Chirale Rhodium-diphosphinkomplexe der allgemeinen Formel

$[Rh(X)(Y)(L_{0,1 \text{ oder } 2})]_{1 \text{ oder } 2}$     I

worin X, welches gegebenenfalls auf einem Träger fixiert sein kann, einen Rest der Formel Z-$COO^-$ darstellt, worin Z wiederum eine Gruppe

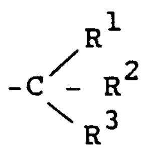 , 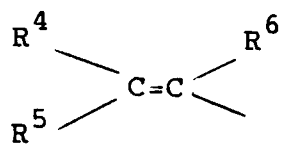

oder Aryl bedeutet, worin

$R^1$, $R^2$, $R^3$ = Wasserstoff, Halogen, niederes Alkyl, Aryl-niederes Alkyl, Perfluor-$C_{1-20}$-Alkyl, Aryl oder die Gruppe -$OR^7$, -$(CH_2)_n$-COA oder AOC-$(CF_2)_n$ bedeuten, wobei wenigstens einer von $R^1$, $R^2$ und $R^3$ -$OR^7$ oder Aryl darstellt,
$R^4$, $R^5$, $R^6$ = Wasserstoff, Halogen, niederes Alkyl, Aryl-niederes Alkyl, Perfluor-$C_{1-20}$-Alkyl, Aryl oder die Gruppe -$(CH_2)_n$-COA oder AOC-$(CF_2)_n$ bedeuten
$R^7$ = Wasserstoff, niederes Alkyl, teilweise oder vollständig halogeniertes niederes Alkyl, Aryl oder Aryl-niederes Alkyl darstellt
A = die Reste -OR oder -$NR'_2$ bedeutet
R = Wasserstoff, niederes Alkyl, Aryl, Aryl-niederes Alkyl oder ein Kation bedeutet,
R' = Wasserstoff, niederes Alkyl, Aryl oder Aryl-niederes Alkyl bedeutet,
n = eine Zahl 0 bis 20 bedeutet,
ferner Y einen chiralen Diphosphinliganden und L einen neutralen Liganden darstellen,

mit Ausnahme derjenigen Komplexe der Formel I, worin X, welches gegebenenfalls auf einem Träger fixiert sein kann, einen Rest der Formel Z-$COO^-$ darstellt, worin Z wiederum die Gruppe

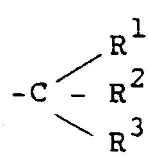

Perfluorphenyl, Perfluorbiphenyl oder einen Rest der Formel

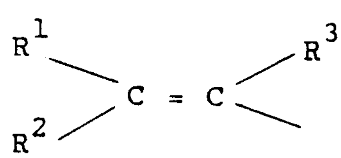

bedeutet und $R^1$, $R^2$ und $R^3$ Halogen, niederes Alkyl, Perfluorphenyl, Perfluor-$C_{1-20}$-Alkyl, Wasserstoff oder die Gruppe -COA oder AOC-$(CF_2)_n$- darstellen. worin A die Reste -OR oder -$NR'_2$ bedeutet, wobei jedoch wenigstens einer der Substituenten $R^1$, $R^2$ und $R^3$ Fluor bedeutet, R Wasserstoff, niederes Alkyl oder ein Kation, R' Wasserstoff oder niederes Alkyl, und n eine Zahl 1 bis 20 bedeuten, wobei die niederen Alkylreste 1-9 Kohlenstoffatome aufweisen und der Ausdruck Aryl für gegebenenfalls substituierte aromatische Kohlenwasserstoffe oder aromatische Heterocyclen mit 4-14 Kohlenstoffatomen steht.

**2.** Chirale Komplexe gemäss Anspruch 1, worin Z die Gruppe

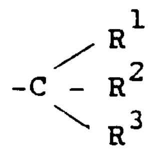

darstellt und $R^1$, $R^2$ und $R^3$ die in Anspruch 1 angegebenen Bedeutungen haben.

**3.** Chirale Komplexe gemäss Anspruch 1 oder 2, worin einer der Substituenten $R^1$, $R^2$ und $R^3$ den Rest -$OR^7$ und die beiden anderen Fluor, Wasserstoff, Perfluor-$C_{1-20}$-Alkyl oder Aryl bedeuten und $R^7$ die in Anspruch 1 angegebene Bedeutung hat.

**4.** Chirale Komplexe gemäss Anspruch 1 oder 2, worin einer der Substituenten $R^1$, $R^2$ und $R^3$ Phenyl und die beiden anderen Fluor, Wasserstoff oder Perfluor-$C_{1-20}$-Alkyl darstellen, wobei jedoch wenigstens einer davon Fluor bedeutet.

**5.** Chirale Komplexe gemäss einem der Ansprüche 1 bis 4, worin Y in der Formel I einen chiralen Liganden der allgemeinen Formel

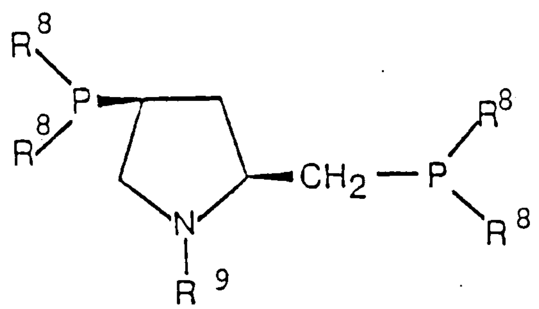 II

darstellt, worin worin $R^8$ Aryl und $R^9$ die Gruppen -CO-$R^{10}$, -$SO_2$-$R^{10}$, -$PO(R^{10})_2$ oder -$PS(R^{10})_2$ darstellen, wobei $R^{10}$ Aryl, niederes Alkyl, di-Arylamino, di-niederes Alkylamino, Aryloxy oder niederes Alkoxy bedeuten, wobei die niederen Alkyl- bzw. Alkoxyreste 1-9 Kohlenstoffatome, aufweisen und der Ausdruck Aryl für sich oder in Aryloxy für gegebenenfalls substituiertes Phenyl steht.

**6.** Chirale Komplexe gemäss Anspruch 5, worin $R^9$ die Gruppe -$PO(R^{10})_2$ darstellt.

**7.** Chirale Komplexe gemäss Anspruch 5 oder 6, worin $R^8$ Phenyl, p-Tolyl, m-Tolyl oder 3,5-Xylyl und $R^{10}$ Phenyl, p-Tolyl, m-Tolyl, p-nied, Alkoxycarbonylphenyl oder tert. Butoxy bedeuten.

**8.** Verfahren zur Herstellung von chiralen Rhodium-diphosphinkomplexen der allgemeinen Formel

$[Rh(X)(Y)(L_{0,1\ oder\ 2})_{1\ oder\ 2}$ I

worin X, welches gegebenenfalls auf einem Träger fixiert sein kann, einen Rest der Formel Z-$COO^-$ darstellt, worin Z wiederum eine Gruppe

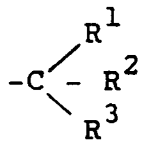 , 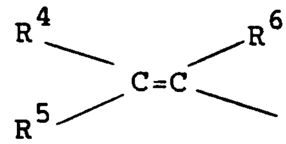

oder Aryl bedeutet, worin

$R^1$, $R^2$, $R^3$ = Wasserstoff, Halogen, niederes Alkyl, Aryl-niederes Alkyl, Perfluor-$C_{1-20}$-Alkyl, Aryl oder die Gruppe $-OR^7$, $-(CH_2)_n$-COA oder AOC-$(CF_2)_n$ bedeuten, wobei wenigstens einer von $R^1$, $R^2$ und $R^3$ $-OR^7$ oder Aryl darstellt,

$R^4$, $R^5$, $R^6$ = Wasserstoff, Halogen, niederes Alkyl, Aryl-niederes Alkyl, Perfluor-$C_{1-20}$-Alkyl, Aryl oder die Gruppe $-(CH_2)_n$-COA oder AOC-$(CF_2)_n$ bedeuten

$R^7$ = Wasserstoff, niederes Alkyl, teilweise oder vollständig halogeniertes niederes Alkyl, Aryl oder Aryl-niederes Alkyl darstellt

A = die Reste -OR oder $-NR'_2$ bedeutet

R = Wasserstoff, niederes Alkyl, Aryl, Aryl-niederes Alkyl oder ein Kation bedeutet,

R' = Wasserstoff, niederes Alkyl, Aryl oder Aryl-niederes Alkyl bedeutet,

n = eine Zahl 0 bis 20 bedeutet,

ferner Y einen chiralen Diphosphinliganden und L einen neutralen Liganden darstellen,

mit Ausnahme derjenigen Komplexe der Formel I, worin X, welches gegebenenfalls auf einem Träger fixiert sein kann, einen Rest der Formel Z-$COO^-$ darstellt, worin Z wiederum die Gruppe

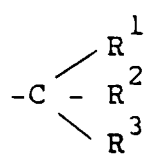

Perfluorphenyl, Perfluorbiphenyl oder einen Rest der Formel

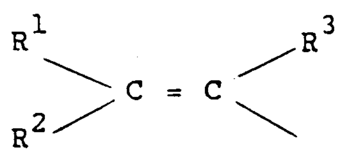

bedeutet und $R^1$, $R^2$ und $R^3$ Halogen, niederes Alkyl, Perfluorphenyl, Perfluor-$C_{1-20}$-Alkyl, Wasserstoff oder die Gruppe -COA oder AOC-$(CF_2)_n$- darstellen, worin A die Reste -OR oder $-NR'_2$ bedeutet, wobei jedoch wenigstens einer der Substituenten $R^1$, $R^2$ und $R^3$ Fluor bedeutet, R Wasserstoff, niederes Alkyl oder ein Kation, R' Wasserstoff oder niederes Alkyl, und n eine Zahl 1 bis 20 bedeuten, wobei die niederen Alkylreste 1-9 Kohlenstoffatome aufweisen und der Ausdruck Aryl für gegebenenfalls substituierte aromatische Kohlenwasserstoffe oder aromatische Heterocyclen mit 4-14 Kohlenstoffatomen steht. dadurch gekennzeichnet, dass man

a) einen Rhodiumkomplex der Formel

$[Rh (X) (L_m)]_{1 \text{ oder } 2}$ III

worin X und L obige Bedeutung haben, und m eine Zahl von 1 bis 4 bedeutet, mit einem chiralen Diphosphinliganden umsetzt, oder

b) einen Rhodiumkomplex der Formel

$[Rh (L_{m+1})]^+ A^-$ IV

worin L und m obige Bedeutung haben und $A^-$ ein Anion, insbesondere $BF_4^-$, $ClO_4^-$, $PF_6^-$ oder

$B(C_6H_5)_4^-$ darstellt,
mit einem chiralen Diphosphinliganden und einem das Anion X enthaltenden Salz umsetzt, oder
c) einen Rhodium-diphosphinkomplex der Formel

$[Rh\ (L_p)\ (Y)]^+\ A^-$ V

worin L, Y und $A^-$ obige Bedeutung haben und p eine Zahl von 1 bis 3 darstellt,
mit einem das Anion X enthaltenden Salz umsetzt, oder
d) einen chiralen Rhodium-diphosphinkomplex der Formel

$[Rh\ (X^1)\ (L_{1\ oder\ 2})\ (Y)]$ VI

worin $X^1$ Halogen bedeutet und L und Y obige Bedeutung haben,
mit einem Silbersalz oder Thalliumsalz der Formel

Ag - X oder Tl - X VII

worin X obige Bedeutung hat,
umsetzt.

**9.** Chirale Rhodium-diphosphinkomplexe der allgemeinen Formel I gemäss den Ansprüchen 1-7, erhältlich gemäss dem Verfahren nach Anspruch 8.

**10.** Verwendung der chiralen Rhodium-diphosphinkomplexe der Formel I gemäss den Ansprüchen 1-7, als Katalysatoren bei asymmetrischen Hydrierungen.

**Patentansprüche für folgenden Vertragsstaat : AT**

**1.** Verfahren zur Herstellung von chiralen Rhodium-diphosphinkomplexen der allgemeinen Formel

$[Rh(X)(Y)(L_{0,1\ oder\ 2})]_{1\ oder\ 2}$ I

worin X, welches gegebenenfalls auf einem Träger fixiert sein kann, einen Rest der Formel Z-$COO^-$ darstellt, worin Z wiederum eine Gruppe

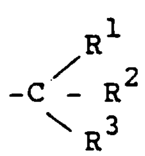 , 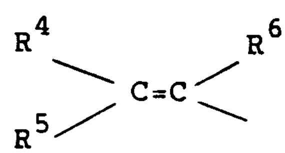

oder Aryl bedeutet, worin

| | |
|---|---|
| $R^1$, $R^2$, $R^3$ = | Wasserstoff, Halogen, niederes Alkyl, Aryl-niederes Alkyl, Perfluor-$C_{1-20}$-Alkyl, Aryl oder die Gruppe -$OR^7$, -$(CH_2)_n$-COA oder AOC-$(CF_2)_n$ bedeuten, wobei wenigstens einer von $R^1$, $R^2$ und $R^3$ -$OR^7$ oder Aryl darstellt, |
| $R^4$, $R^5$, $R^6$ = | Wasserstoff, Halogen, niederes Alkyl, Aryl-niederes Alkyl, Perfluor-$C_{1-20}$-Alkyl, Aryl oder die Gruppe -$(CH_2)_n$-COA oder AOC-$(CF_2)_n$ bedeuten |
| $R^7$ = | Wasserstoff, niederes Alkyl, teilweise oder vollständig halogeniertes niederes Alkyl, Aryl oder Aryl-niederes Alkyl darstellt |
| A = | die Reste -OR oder -$NR'_2$ bedeutet |
| R = | Wasserstoff, niederes Alkyl, Aryl, Aryl-niederes Alkyl oder ein Kation bedeutet, |
| R' = | Wasserstoff, niederes Alkyl, Aryl oder Aryl-niederes Alkyl bedeutet, |
| n = | eine Zahl 0 bis 20 bedeutet, ferner Y einen chiralen Diphosphinliganden und L einen neutralen Liganden darstellen, |

mit Ausnahme derjenigen Komplexe der Formel I, worin X, welches gegebenenfalls auf einem Träger

fixiert sein kann, einen Rest der Formel Z-COO⁻ darstellt, worin Z wiederum die Gruppe

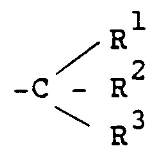

Perfluorphenyl, Perfluorbiphenyl oder einen Rest der Formel

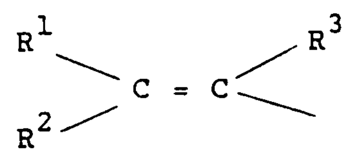

bedeutet und $R^1$, $R^2$ und $R^3$ Halogen, niederes Alkyl, Perfluorphenyl, Perfluor-$C_{1-20}$-Alkyl, Wasserstoff oder die Gruppe -COA oder AOC-$(CF_2)n^-$ darstellen, worin A die Reste -OR oder -NR'$_2$ bedeutet, wobei jedoch wenigstens einer der Substituenten $R^1$, $R^2$ und $R^3$ Fluor bedeutet, R Wasserstoff, niederes Alkyl oder ein Kation, R' Wasserstoff oder niederes Alkyl, und n eine Zahl 1 bis 20 bedeuten, wobei die niederen Alkylreste 1-9 Kohlenstoffatome aufweisen und der Ausdruck Aryl für gegebenenfalls substituierte aromatische Kohlenwasserstoffe oder aromatische Heterocyclen mit 4-14 Kohlenstoffatomen steht.
dadurch gekennzeichnet, dass man

a) einen Rhodiumkomplex der Formel

$[Rh\ (X)\ (L_m)]_{1\ oder\ 2}$ III

worin X und L obige Bedeutung haben, und m eine Zahl von 1 bis 4 bedeutet,
mit einem chiralen Diphosphinliganden umsetzt, oder

b) einen Rhodiumkomplex der Formel

$[Rh\ (L_{m+1})]^+\ A^-$ IV

worin L und m obige Bedeutung haben und $A^-$ ein Anion, insbesondere $BF_4^-$, $ClO_4^-$, $PF_6^-$ oder $B(C_6H_5)_4^-$ darstellt,
mit einem chiralen Diphosphinliganden und einem das Anion X enthaltenden Salz umsetzt, oder

c) einen Rhodium-diphosphinkomplex der Formel

$[Rh\ (L_p)\ (Y)]^+\ A^-$ V

worin L, Y und $A^-$ obige Bedeutung haben und p eine Zahl von 1 bis 3 darstellt,
mit einem das Anion X enthaltenden Salz umsetzt, oder

d) einen chiralen Rhodium-diphosphinkomplex der Formel

$[Rh\ (X^1)\ (L_{1\ oder\ 2})\ (Y)]$ VI

worin $X^1$ Halogen bedeutet und L und Y obige Bedeutung haben,
mit einem Silbersalz oder Thalliumsalz der Formel

Ag - X oder Tl - X VII

worin X obige Bedeutung hat,

umsetzt.

**2.** Verfahren gemäss Anspruch 1, dadurch gekennzeichnet, dass man Ausgangsmaterialien verwendet,

worin Z in dem Rest X die Gruppe

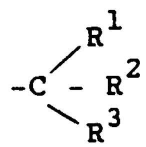

bedeutet.

3. Verfahren gemäss Anspruch 1 oder 2, dadurch gekennzeichnet, dass man Ausgangsmaterialien verwendet, worin einer der Substituenten $R^1$, $R^2$ und $R^3$ den Rest $-OR^7$ und die beiden anderen Fluor, Wasserstoff, Perfluor-$C_{1-20}$-Alkyl oder Aryl bedeuten und $R^7$ die in Anspruch 1 angegebene Bedeutung hat.

4. Verfahren gemäss einem der Ansprüche 1 bis 3, dadurch gekennzeichnet, dass man Ausgangsmaterialien verwendet, worin einer der Substituenten $R^1$, $R^2$ und $R^3$ Phenyl und die beiden anderen Fluor, Wasserstoff oder Perfluor-$C_{1-20}$-Alkyl darstellen, wobei jedoch wenigstens einer davon Fluor bedeutet.

5. Verfahren gemäss einem der Ansprüche 1 bis 4, dadurch gekennzeichnet, dass der Diphosphinligand Y einen chiralen Liganden der allgemeinen Formel

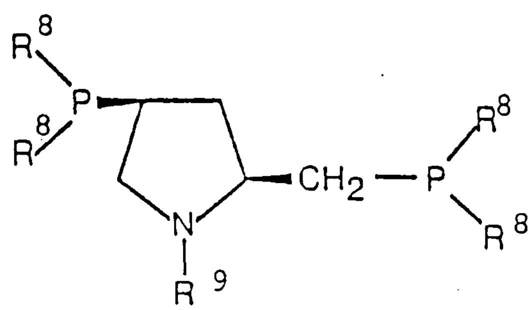

II

darstellt, worin worin $R^8$ Aryl und $R^9$ die Gruppen $-CO-R^{10}$, $-SO_2-R^{10}$, $-PO(R^{10})_2$ oder $-PS(R^{10})_2$ darstellen, wobei $R^{10}$ Aryl, niederes Alkyl, di-Arylamino, di-niederes Alkylamino, Aryloxy oder niederes Alkoxy bedeuten, wobei die niederen Alkyl- bzw. Alkoxyreste 1-9 Kohlenstoffatome aufweisen und der Ausdruck Aryl für sich oder in Aryloxy für gegebenenfalls substituiertes Phenyl steht.

6. Verfahren gemäss Anspruch 5, dadurch gekennzeichnet, dass in dem Liganden der Formel II $R^9$ die Gruppe $-PO(R^{10})_2$ darstellt.

7. Verfahren gemäss Anspruch 5 oder 6, dadurch gekennzeichnet, dass in dem Liganden der Formel II $R^8$ Phenyl, p-Tolyl, m-Tolyl oder 3,5-Xylyl und $R^{10}$ Phenyl, p-Tolyl, m-Tolyl, p-nied. Alkoxycarbonylphenyl oder tert. Butoxy bedeuten.

8. Verwendung der gemäss den Ansprüchen 1-7 herstellbaren chiralen Rhodiumdiphosphinkomplexe der Formel I, als Katalysatoren bei asymmetrischen Hydrierungen.

9. Verwendung der gemäss den Ansprüchen 1-7 herstellbaren chiralen Rhodiumdiphosphinkomplexe der Formel I, bei der asymmetrischen Hydrierung von Ketopantolacton zu Pantolacton.

**Claims**

**Claims for the following Contracting States : BE, CH, DE, FR, GB, IT, LI, NL**

1. Chiral rhodium-diphosphine complexes of the general formula

$[Rh(X)(Y)(L_{0,1\ or\ 2})]_{1\ or\ 2}$ I

wherein X, which can be optionally fixed to a carrier, represents a residue of the formula Z-COO⁻ in which Z signifies a group

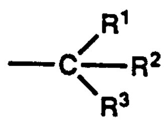 , 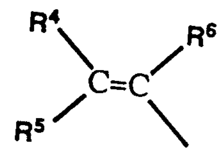

or aryl, wherein

$R^1$, $R^2$, $R^3$ = hydrogen, halogen, lower alkyl, aryl-lower alkyl, perfluoro-$C_{1-20}$-alkyl, aryl or the group $-OR^7$, $-(CH_2)_n$-COA or AOC-$(CF_2)_n$, whereby at least one of $R^1$, $R^2$ and $R^3$ represents $-OR^7$ or aryl,

$R^4$, $R^5$, $R^6$ = hydrogen, halogen, lower alkyl, aryl-lower alkyl, perfluoro-$C_{1-20}$-alkyl, aryl or the group $-(CH_2)_n$-COA or AOC-$(CF_2)_n$,

$R^7$ = represents hydrogen, lower alkyl, partially or completely halogenated lower alkyl, aryl or aryl-lower alkyl,

A = the residue -OR or -NR'$_2$,

R = hydrogen, lower alkyl, aryl, aryl-lower alkyl or a cation,

R' = hydrogen, lower alkyl, aryl or aryl-lower alkyl and

n = a number 0 to 20, and wherein

further Y represents a chiral diphosphine ligand and L represents a neutral ligand,

with the exception of those complexes of formula I in which X, which can be optionally fixed to a carrier, represents a residue of the formula Z-COO⁻ in which Z signifies the group

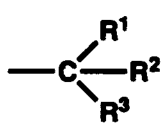 ,

perfluorophenyl, perfluorobiphenyl or a residue of the formula

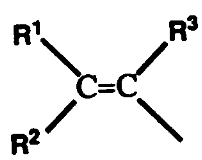

and $R^1$, $R^2$ and $R^3$ represent halogen, lower alkyl, perfluorophenyl, perfluoro-$C_{1-20}$-alkyl, hydrogen or the group -COA or AOC-$(CF_2)_n$- in which A signifies the residue -OR or -NR'$_2$, whereby, however, at least one of the substituents $R^1$, $R^2$ and $R^3$ signifies fluorine, R signifies hydrogen, lower alkyl or a cation, R' signifies hydrogen or lower alkyl and n signifies a number 1 to 20, whereby the lower alkyl residues have 1-9 carbon atoms and the term aryl stands for optionally substituted aromatic hydrocarbons or aromatic heterocycles with 4-14 carbon atoms.

**2.** Chiral complexes in accordance with claim 1, wherein Z represents the group

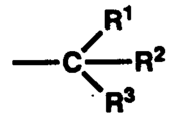

and $R^1$, $R^2$ and $R^3$ have the significances given in claim 1.

**3.** Chiral complexes in accordance with claim 1 or 2, wherein one of the substituents $R^1$, $R^2$ and $R^3$ signifies the residue $-OR^7$ and the other two signify fluorine, hydrogen, perfluoro-$C_{1-20}$-alkyl or aryl and $R^7$ has the significance given in claim 1.

**4.** Chiral complexes in accordance with claim 1 or 2, wherein one of the substituents $R^1$, $R^2$ and $R^3$ represents phenyl and the other two represent fluorine, hydrogen or perfluoro-$C_{1-20}$-alkyl, whereby, however, at least one of them signifies fluorine.

**5.** Chiral complexes in accordance with any one of claims 1 to 4, wherein Y in formula I represents a chiral ligand of the general formula

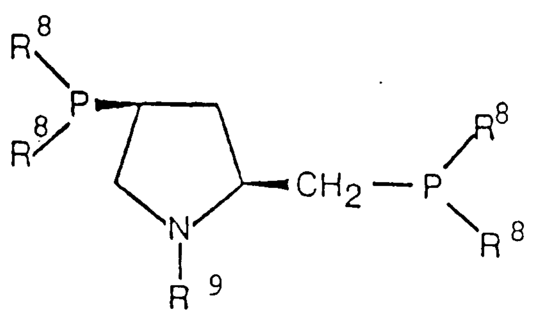

II

wherein $R^8$ represents aryl and $R^9$ represents the group $-CO-R^{10}$, $-SO_2-R^{10}$, $-PO(R^{10})_2$ or $-PS(R^{10})_2$ in which $R^{10}$ signifies aryl, lower alkyl, di-arylamino, di-lower alkylamino, aryloxy or lower alkoxy, whereby the lower alkyl and, respectively, lower alkoxy residues have 1-9 carbon atoms and the term aryl per se or in aryloxy stands for optionally substituted phenyl.

**6.** Chiral complexes in accordance with claim 5, wherein $R^9$ represents the group $-PO(R^{10})_2$.

**7.** Chiral complexes in accordance with claim 5 or 6, wherein $R^8$ signifies phenyl, p-tolyl, m-tolyl or 3,5-xylyl and $R^{10}$ signifies phenyl, p-tolyl, m-tolyl,, p-lower alkoxycarbonylphenyl or tert.butoxy

**8.** A process for the manufacture of chiral rhodium-diphosphine complexes of the general formula

$$[Rh(X)(Y)(L_{0,1 \text{ or } 2})]_{1 \text{ or } 2} \qquad I$$

wherein X, which can be optionally fixed to a carrier, represents a residue of the formula $Z-COO^-$ in which Z signifies a group

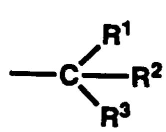

,

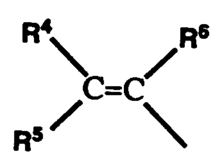

or aryl, wherein

$R^1$, $R^2$, $R^3$ = hydrogen, halogen, lower alkyl, aryl-lower alkyl, perfluoro-$C_{1-20}$-alkyl, aryl or the

group $-OR^7$, $-(CH_2)_n-COA$ or $AOC-(CF_2)_n$, whereby at least one of $R^1$, $R^2$ and $R^3$ represents $-OR^7$ or aryl,

$R^4$, $R^5$, $R^6$ = hydrogen, halogen, lower alkyl, aryl-lower alkyl, perfluoro-$C_{1-20}$-alkyl, aryl or the group $-(CH_2)_n-COA$ or $AOC-(CF_2)_n$,

$R^7$ = represents hydrogen, lower alkyl, partially or completely halogenated lower alkyl, aryl or aryl-lower alkyl,

A = the residue -OR or $-NR'_2$,

R = hydrogen, lower alkyl, aryl, aryl-lower alkyl or a cation,

R' = hydrogen, lower alkyl, aryl or aryl-lower alkyl and

n = a number 0 to 20, and wherein further Y represents a chiral diphosphine ligand and L represents a neutral ligand,

with the exception of those complexes of formula I in which X, which can be optionally fixed to a carrier, represents a residue of the formula $Z-COO^-$ in which Z signifies the group

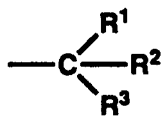

perfluorophenyl, perfluorobiphenyl or a residue of the formula

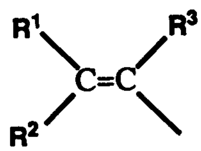

and $R^1$, $R^2$ and $R^3$ represent halogen, lower alkyl, perfluorophenyl, perfluoro-$C_{1-20}$-alkyl, hydrogen or the group -COA or $AOC-(CF_2)_n$- in which A signifies the residue -OR or $-NR'_2$, whereby, however, at least one of the substituents $R^1$, $R^2$ and $R^3$ signifies fluorine, R signifies hydrogen, lower alkyl or a cation, R' signifies hydrogen or lower alkyl and n signifies a number 1 to 20, whereby the lower alkyl residues have 1-9 carbon atoms and the term aryl stands for optionally substituted aromatic hydrocarbons or aromatic heterocycles with 4-14 carbon atoms,

characterized by

a) reacting a rhodium complex of the formula

$[Rh (X) (L_m)]_{1 \text{ or } 2}$ III

wherein X and L have the above significance and m signifies a number of 1 to 4,
with a chiral diphosphine ligand, or

b) reacting a rhodium complex of the formula

$[Rh(L_{m+1})]^+ A^-$ IV

wherein L and m have the above significance and $A^-$ represents an anion, especially $BF_4^-$, $ClO_4^-$, $PF_6^-$ or $B(C_6H_5)_4^-$,
with a chiral diphosphine ligand and a salt containing the anion X, or

c) reacting a rhodium-diphosphine complex of the formula

$[Rh (L_p) (Y)]^+ A^-$ V

wherein L, Y and $A^-$ have the above significance and p represents a number of 1 to 3,
with a salt containing the anion X, or

d) reacting a chiral rhodium-diphosphine complex of the formula

$[Rh (X^1) (L_{1 \text{ or } 2}) (Y)]$ VI

wherein $X^1$ signifies halogen and L and Y have the above significance, with a silver salt or thallium salt of the formula

Ag-X or Tl-X VII

wherein X has the above significance.

**9.** Chiral rhodium-diphosphine complexes of general formula I in accordance with claims 1-7, obtainable in accordance with the process according to claim 8.

**10.** The use of the chiral rhodium-diphosphine complexes of formula I in accordance with claims 1-7 as catalysts in asymmetric hydrogenations.

**Claims for the following Contracting State : AT**

**1.** A process for the manufacture of chiral rhodium-diphosphine complexes of the general formula

$[Rh(X)(Y)(L_{0,1 \text{ or } 2})]_{1 \text{ or } 2}$ I

wherein X, which can be optionally fixed to a carrier, represents a residue of the formula Z-COO⁻ in which Z signifies a group

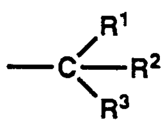

,

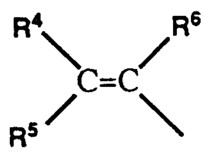

or aryl, wherein

| | |
|---|---|
| $R^1$, $R^2$, $R^3$ = | hydrogen, halogen, lower alkyl, aryl-lower alkyl, perfluoro-$C_{1-20}$-alkyl, aryl or the group -OR , -$(CH_2)_n$-COA or AOC-$(CF_2)_n$, whereby at least one of $R^1$, $R^2$ and $R^3$ represents -$OR^7$ or aryl, |
| $R^4$, $R^5$, $R^6$ = | hydrogen, halogen, lower alkyl, aryl-lower alkyl, perfluoro-$C_{1-20}$-alkyl, aryl or the group -$(CH_2)_n$-COA or AOC-$(CF_2)_n$, |
| $R^7$ = | represents hydrogen, lower alkyl, partially or completely halogenated lower alkyl, aryl or aryl-lower alkyl, |
| A = | the residue -OR or -NR'$_2$, |
| R = | hydrogen, lower alkyl, aryl, aryl-lower alkyl or a cation, |
| R' = | hydrogen, lower alkyl, aryl or aryl-lower alkyl and |
| n = | a number 0 to 20, and wherein |

further Y represents a chiral diphosphine ligand and L represents a neutral ligand, with the exception of those complexes of formula I in which X, which can be optionally fixed to a carrier, represents a residue of the formula Z-COO⁻ in which Z signifies the group

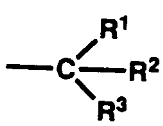

,

perfluorophenyl, perfluorobiphenyl or a residue of the formula

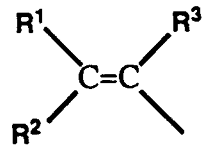

and $R^1$, $R^2$ and $R^3$ represent halogen, lower alkyl, perfluorophenyl, perfluoro-$C_{1-20}$-alkyl, hydrogen or the group -COA or AOC-$(CF_2)_n$- in which A signifies the residue -OR or -NR'$_2$, whereby, however, at least one of the substituents $R^1$, $R^2$ and $R^3$ signifies fluorine, R signifies hydrogen, lower alkyl or a cation, R' signifies hydrogen or lower alkyl and n signifies a number 1 to 20, whereby the lower alkyl residues have 1-9 carbon atoms and the term aryl stands for optionally substituted aromatic hydrocarbons or aromatic heterocycles with 4-14 carbon atoms.
characterized by

a) reacting a rhodium complex of the formula

$[Rh (X) (L_m)]_{1 \text{ or } 2}$ III

wherein X and L have the above significance and m signifies a number of 1 to 4, with a chiral diphosphine ligand, or

b) reacting a rhodium complex of the formula

$[Rh(L_{m+1})]^+ A^-$ IV

wherein L and m have the above significance and $A^-$ represents an anion, especially $BF_4^-$, $ClO_4^-$, $PF_6^-$ or $B(C_6H_5)_4^-$, with a chiral diphosphine ligand and a salt containing the anion X, or

c) reacting a rhodium-diphosphine complex of the formula

$[Rh (L_p) (Y)]^+ A^-$ V

wherein L, Y and $A^-$ have the above significance and p represents a number of 1 to 3, with a salt containing the anion X, or

d) reacting a chiral rhodium-diphosphine complex of the formula

$[Rh (X^1) (L_{1 \text{ or } 2}) (Y)]$ VI

wherein $X^1$ signifies halogen and L and Y have the above significance, with a silver salt or thallium salt of the formula

Ag-X or Tl-X VII

wherein X has the above significance.

**2.** A process in accordance with claim 1, characterized in that starting materials in which Z represents the group

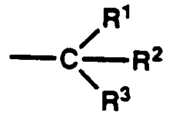

and $R^1$, $R^2$ and $R^3$ have the significances given in claim 1 are used.

**3.** A process in accordance with claim 1 or 2, wherein starting materials in which one of the substituents $R^1$, $R^2$ and $R^3$ signifies the residue -$OR^7$ and the other two signify fluorine, hydrogen, perfluoro-$C_{1-20}$-alkyl or aryl and $R^7$ has the significance given in claim 1 are used.

**4.** A process in accordance with claim 1, 2 or 3, characterized in that starting materials in which one of the substituents $R^1$, $R^2$ and $R^3$ represents phenyl and the other two represent fluorine, hydrogen or perfluoro-$C_{1-20}$-alkyl, whereby, however, at least one of them signifies fluorine, are used.

**5.** A process in accordance with any one of claims 1 to 4, characterized in that the diphosphine ligand Y in formula I represents a chiral ligand of the general formula

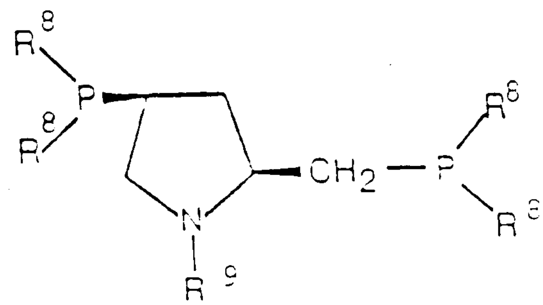

II

wherein $R^8$ represents aryl and $R^9$ represents the group $-CO-R^{10}$, $-SO_2-R^{10}$, $-PO(R^{10})_2$ or $-PS(R^{10})_2$ in which $R^{10}$ signifies aryl, lower alkyl, di-arylamino, di-lower alkylamino, aryloxy or lower alkoxy, whereby the lower alkyl and, respectively, lower alkoxy residues have 1-9 carbon atoms and the term aryl per se or in aryloxy stands for optionally substituted aromatic hydrocarbons or aromatic heterocycles with 4-14 carbon atoms.

**6.** A process in accordance with claim 5, characterized in that $R^9$ in the ligand of formula II represents the group $-PO(R^{10})_2$.

**7.** A process in accordance with claim 5 or 6, characterized in that in the ligands of formula II $R^8$ signifies phenyl, p-tolyl, m-tolyl or 3,5-xylyl and $R^{10}$ signifies phenyl, p-tolyl, m-tolyl, p-lower alkoxycarbonylphenyl or tert.butoxy.

**8.** The use of the chiral rhodium-diphosphine complexes of formula I manufacturable in accordance with claims 1-7 as catalysts in asymmetric hydrogenations.

**9.** The use of the chiral rhodium diphosphine complexes manufacturable in accordance with claims 1-7 for the asymmetric hydrogenation of ketopantolactone to pantolactone.

**Revendications**

**Revendications pour les Etats contractants suivants : BE, CH, DE, FR, GB, IT, LI, NL**

**1.** Complexes chiraux de rhodium et de diphosphines de formule générale

$[Rh(X)(Y)(L_{0,\,1\ ou\ 2})]_{1\ ou\ 2}$ I

où X qui peut être éventuellement fixé sur un support, représente un reste de formule $Z-COO^-$, Z représentant de nouveau un groupe

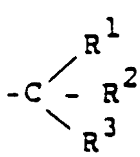

,

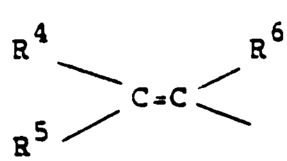

ou un aryle,

$R^1$, $R^2$, $R^3$ représentant l'hydrogène, un halogène, un alkyle inférieur, un arylalkyle inférieur, un perfluoroalkyle en $C_{1-20}$, un aryle ou le groupe $-OR^7$, $-(CH_2)_n-COA$ ou $AOC-(CF_2)_n$,

au moins l'un des radicaux $R^1$, $R^2$ et $R^3$ représentant $-OR^7$ ou un aryle,

$R^4$, $R^5$, $R^6$ représentant l'hydrogène, un halogène, un alkyle inférieur ou un arylalkyle inférieur, un perfluoroalkyle en $C_{1-20}$, un aryle ou le groupe $-(CH_2)_n$-COA ou AOC-$(CF_2)_n$,

$R^7$ représentant l'hydrogène, un alkyle inférieur, un alkyle inférieur partiellement ou totalement halogéné, un aryle ou un arylalkyle inférieur,

A représentant les restes -OR ou -NR'$_2$,

R représentant l'hydrogène, un alkyle inférieur, un aryle, un arylalkyle inférieur ou un cation,

R' représentant l'hydrogène, un alkyle inférieur, un aryle ou un arylalkyle inférieur,

n représentant un nombre allant de 0 à 20,

Y représentant en outre, un ligand diphosphine chiral et L représentant un ligand neutre,

à l'exception des complexes de formule I où X qui peut être éventuellement fixé sur un support, représente un reste de formule Z-$COO^-$, Z représentant de nouveau le groupe

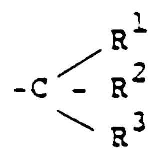

le perfluorophényle, le perfluorobiphényle ou un reste de formule

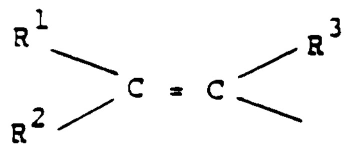

et $R^1$, $R^2$ et $R^3$ représentent un halogène, un alkyle inférieur, le perfluorophényle, un perfluoroalkyle en $C_{1-20}$, l'hydrogène ou le groupe -COA ou AOC-$(CF)_2$n-,A représentant le reste -OR ou NR'$_2$, l'un des substituants $R^1$, $R^2$ et $R^3$ au moins représentant toutefois le fluor, R représentant l'hydrogène, un alkyle inférieur ou un cation, R' représentant l'hydrogène ou un alkyle inférieur et n un nombre allant de 1 à 20, les restes alkyles inférieurs présentant 1 à 9 atomes de carbone et l'expression aryle signifiant des hydrocarbures aromatiques ou des hétérocycles aromatiques ayant 4 à 14 atomes de carbone, éventuellement substitués.

**2.** Complexes chiraux selon la revendication 1, où Z représente le groupe

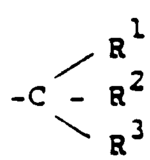

et $R^1$, $R^2$ et $R^3$ ont les significations indiquées dans la revendication 1.

**3.** Complexes chiraux selon la revendication 1 ou 2, où l'un des substituants $R^1$, $R^2$ et $R^3$ représente le reste $-OR^7$ et les deux autres le fluor, l'hydrogène, un perfluoroalkyle en $C_{1-20}$ ou un aryle et $R^7$ a la signification indiquée dans la revendication 1.

**4.** Complexes chiraux selon la revendication 1 ou 2, où l'un des substituants $R^1$, $R^2$ et $R^3$ représente le phényle et les deux autres le fluor, l'hydrogène ou un perfluoroalkyle en $C_{1-20}$, l'un au moins des substituants représentant toutefois le fluor.

**5.** Complexes chiraux selon l'une des revendications 1 à 4, où Y, dans la formule I, représente un ligand chiral de formule générale

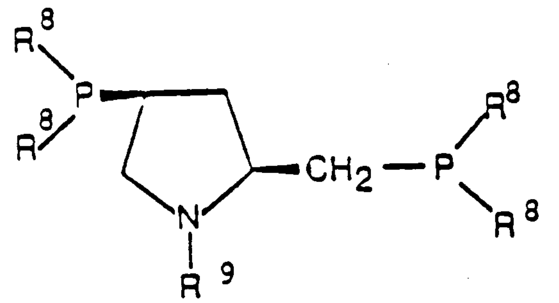

II

$R^8$ représentant un aryle et $R^9$ représentant les groupes $-CO-R^{10}$, $-SO_2-R^{10}$, $-PO(R^{10})_2$ ou $-PS(R^{10})_2$, $R^{10}$ représentant un aryle, un alkyle inférieur, un diarylamino, un dialkyle inférieur amino, un aryloxy ou un alcoxy inférieur, les restes alkyles inférieurs ou alcoxy inférieurs présentant 1 à 9 atomes de carbone et l'expression aryle prise en soi ou dans aryloxy représentant le phényle éventuellement substitué.

**6.** Complexes chiraux selon la revendication 5, où $R^9$ représente le groupe $-PO(R^{10})_2$.

**7.** Complexes chiraux selon la revendication 5 ou 6, où $R^8$ représente le phényle, le p-tolyle, le m-tolyle ou le 3,5-xylyle et $R^{10}$ représente le phényle, le p-tolyle, le m-tolyle, un p-alcoxy inférieur carbonylphényle ou le tert.butoxy.

**8.** Procédé pour la préparation des complexes chiraux de rhodium et de diphosphines de formule générale

$[Rh(X)(Y)(L_{0,\ 1\ ou\ 2})]_{1\ ou\ 2}$ I

où X qui peut être éventuellement fixé sur un support, représente un reste de formule $Z-COO^-$, Z représentant de nouveau un groupe

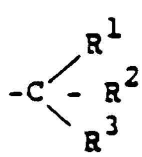

,

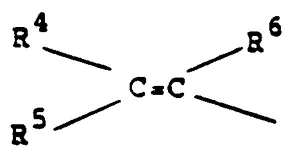

ou un aryle,

| | |
|---|---|
| $R^1$, $R^2$, $R^3$ | représentant l'hydrogène, un halogène, un alkyle inférieur, un alkylaryle inférieur, un perfluoroalkyle en $C_{1-20}$, un aryle ou le groupe $-OR^7$, $-(CH_2)_n-COA$ ou $AOC-(CF_2)_n$, au moins l'un des radicaux $R^1$, $R^2$ et $R^3$ représentant $-OR^7$ ou un aryle, |
| $R^4$, $R^5$, $R^6$ | représentant l'hydrogène, un halogène, un alkyle inférieur ou un arylalkyle inférieur, un perfluoroalkyle en $C_{1-20}$, un aryle ou le groupe $-(CH_2)_n-COA$ ou $AOC-(CF_2)_n$, |
| $R^7$ | représentant l'hydrogène, un alkyle inférieur, un alkyle inférieur partiellement ou totalement halogéné, un aryle ou un arylalkyle inférieur, |
| A | représentant les restes -OR ou $-NR'_2$, |
| R | représentant l'hydrogène, un alkyle inférieur, un aryle, un arylalkyle inférieur ou un cation, |
| R' | représentant l'hydrogène, un alkyle inférieur, un aryle ou un arylalkyle inférieur, |
| n | représentant un nombre allant de 0 à 20, |
| | Y représentant en outre, un ligand diphosphine chiral et L représentant un ligand neutre, |

à l'exception des complexes de formule I où X qui peut être éventuellement fixé sur un support,

représente un reste de formule Z-$COO^-$, Z représentant de nouveau le groupe

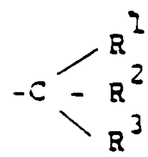

le perfluorophényle, le perfluorobiphényle ou un reste de formule

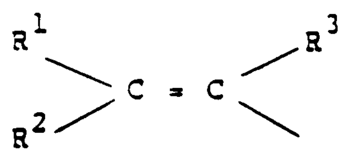

et $R^1$, $R^2$ et $R^3$ représentent un halogène, un alkyle inférieur, le perfluorophényle, un perfluoroalkyle en $C_{1-20}$, l'hydrogène ou le groupe -COA ou AOC-$(CF_2)_n$-, A représentant le reste -OR ou $NR'_2$, l'un des substituants $R^1$, $R^2$ et $R^3$ au moins représentant toutefois le fluor, R représentant l'hydrogène, un alkyle inférieur ou un cation, R' représentant l'hydrogène ou un alkyle inférieur et n un nombre allant de 1 à 20, les restes alkyles inférieurs présentant 1 à 9 atomes de carbone et l'expression aryle signifiant des hydrocarbures aromatiques ou des hétérocycles aromatiques ayant 4 à 14 atomes de carbone, éventuellement substitués,
caractérisé

a) en ce que l'on fait réagir un complexe de rhodium de formule

$[Rh(X)(L_m)]_{1 \text{ ou } 2}$ III

où X et L ont la signification donnée ci-dessus et m représente un nombre allant de 1 à 4, avec un ligand diphosphine chiral ou

b) en ce que l'on fait réagir un complexe de rhodium de formule

$[Rh(L_{m+1})]^+ A^-$ IV

où L et m ont la signification donnée ci-dessus et $A^-$ représente un anion, en particulier $BF_4^-$, $ClO_4^-$, $PF_6^-$ ou $B(C_6H_5)_4^-$, avec un ligand diphosphine chiral et un sel contenant l'anion X ou

c) en ce que l'on fait réagir un complexe de rhodium et de diphosphines de formule

$[Rh(L_p)(Y)]^+ A^-$ V

où L, Y et $A^-$ ont la signification donnée ci-dessus et p représente un nombre allant de 1 à 3, avec un sel contenant l'anion X ou

d) en ce que l'on fait réagir un complexe de rhodium et de diphosphines de formule

$[Rh(X^1)(L_{1 \text{ ou } 2})(Y)]$ VI

où $X^1$ représente un halogène et L et Y ont la signification donnée ci-dessus, avec un sel d'argent ou un sel de thallium de formule

Ag - X ou Tl - X VII

où X a la signification donnée ci-dessus.

**9.** Complexes chiraux de rhodium et de diphosphines de formule générale I selon les revendications 1 à 7

pouvant être obtenus par le procédé selon la revendication 8.

**10.** Utilisation des complexes chiraux de rhodium et de diphosphines de formule I selon les revendications 1 à 7 comme catalyseurs dans les hydrogénations asymétriques.

**Revendications pour l'Etat contractant suivant : AT**

**1.** Procédé pour la préparation des complexes chiraux de rhodium et de diphosphines de formule générale

$[Rh(X)(Y)(L_{0,\ 1\ ou\ 2})]_{1\ ou\ 2}$ I

où X qui peut être éventuellement fixé sur un support, représente un reste de formule Z-COO⁻, Z représentant de nouveau un groupe

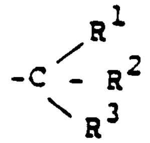

,

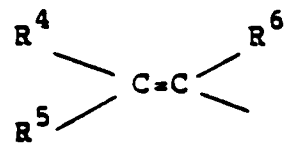

ou un aryle,

| | |
|---|---|
| $R^1$, $R^2$, $R^3$ | représentant l'hydrogène, un halogène, un alkyle inférieur, un arylalkyle inférieur, un perfluoroalkyle en $C_{1-20}$, un aryle ou le groupe $-OR^7$, $-(CH_2)_n$-COA ou AOC-$(CF_2)_n$, au moins l'un des radicaux $R^1$, $R^2$ et $R^3$ représentant $-OR^7$ ou un aryle, |
| $R^4$, $R^5$, $R^6$ | représentant l'hydrogène, un halogène, un alkyle inférieur ou un arylalkyle inférieur, un perfluoroalkyle en $C_{1-20}$, un aryle ou le groupe $-(CH_2)_n$-COA ou AOC-$(CF_2)_n$, |
| $R^7$ | représentant l'hydrogène, un alkyle inférieur, un alkyle inférieur partiellement ou totalement halogéné, un aryle ou un arylalkyle inférieur, |
| A | représentant les restes -OR ou $-NR'_2$, |
| R | représentant l'hydrogène, un alkyle inférieur, un aryle, un arylalkyle inférieur ou un cation, |
| R' | représentant l'hydrogène, un alkyle inférieur, un aryle ou un arylalkyle inférieur, |
| n | représentant un nombre allant de 0 à 20, |

Y représentant, en outre, un ligand diphosphine chiral et L représentant un ligand neutre,

à l'exception des complexes de formule I où X qui peut être éventuellement fixé sur un support, représente un reste de formule Z-COO⁻, Z représentant de nouveau le groupe

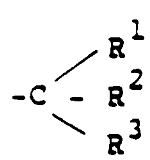

le perfluorophényle, le perfluorobiphényle ou un reste de formule

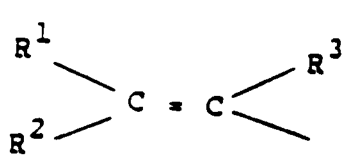

et $R^1$, $R^2$ et $R^3$ représentent un halogène, un alkyle inférieur, le perfluorophényle, un perfluoroalkyle en

$C_{1-20}$, l'hydrogène ou le groupe -COA ou AOC-$(CF_2)_n$- A représentant le reste -OR ou -$NR'_2$, l'un des substituants $R^1$, $R^2$ et $R^3$ au moins représentant toutefois le fluor, R représentant l'hydrogène, un alkyle inférieur ou un cation, R' représentant l'hydrogène ou un alkyle inférieur et n un nombre allant de 1 à 20, les restes alkyles inférieurs présentant 1 à 9 atomes de carbone et l'expression aryle signifiant des hydrocarbures aromatiques ou des hétérocycles aromatiques ayant 4 à 14 atomes de carbone, éventuellement substitués,
caractérisé

a) en ce que l'on fait réagir un complexe de rhodium de formule

$[Rh(X)(L_m)]_{1\ ou\ 2}$ III

où X et L ont la signification donnée ci-dessus et m représente un nombre allant de 1 à 4, avec un ligand diphosphine chiral ou

b) en ce que l'on fait réagir un complexe de rhodium de formule

$[Rh(L_{m+1})]^+ A^-$ IV

où L et m ont la signification donnée ci-dessus et $A^-$ représente un anion, en particulier $BF_4^-$, $ClO_4^-$, $PF_6^-$ ou $B(C_6H_5)_4^-$,
avec un ligand diphosphine chiral et un sel contenant l'anion X ou

c) en ce que l'on fait réagir un complexe de rhodium et de diphosphines de formule

$[Rh(L_p)(Y)]^+ A^-$ V

où L, Y et $A^-$ ont la signification donnée ci-dessus et p représente un nombre allant de 1 à 3, avec un sel contenant l'anion X ou

d) en ce que l'on fait réagir un complexe de rhodium et de diphosphines de formule

$[Rh(X^1)(L_{1\ ou\ 2})(Y)]$ VI

où $X^1$ représente un halogène et L et Y ont la signification donnée ci-dessus,
avec un sel d'argent ou un sel de thallium de formule

Ag - X ou Tl - X VII

où X a la signification donnée ci-dessus.

2. Procédé selon la revendication 1, caractérisé en ce que l'on utilise des produits de départ où dans le reste Z représente le groupe

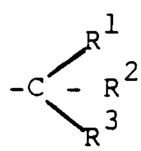

et $R^1$, $R^2$ et $R^3$ ont les significations indiquées dans la revendication 1.

3. Procédé selon la revendication 1 ou 2, caractérisé en ce que l'on utilise des produits de départ où l'un des substituants $R^1$, $R^2$ et $R^3$ représente le reste -$OR^7$ et les deux autres le fluor, l'hydrogène, un perfluoroalkyle en $C_{1-20}$ ou un aryle et $R^7$ a la signification indiquée dans la revendication 1.

4. Procédé selon l'une des revendications 1 à 3, caractérisé en ce que l'on utilise des produits de départ où l'un des substituants $R^1$, $R^2$ et $R^3$ représente le phényle et les deux autres le fluor, l'hydrogène ou un perfluoroalkyle en $C_{1-20}$, l'un au moins des substituants représentant toutefois le fluor.

5. Procédé selon l'une des revendications 1 à 4, caractérisé en ce que le ligand diphosphine Y représente un ligand chiral de formule générale

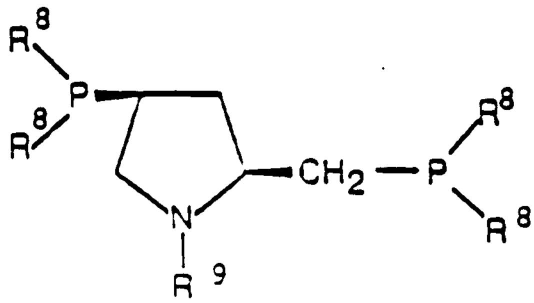

II

$R^8$ représentant un aryle et $R^9$ représentant les groupes $-CO-R^{10}$, $-SO_2-R^{10}$, $-PO(R^{10})_2$ ou $-PS(R^{10})_2$, $R^{10}$ représentant un aryle, un alkyle inférieur, un diarylamino, un dialkyle inférieur amino, un aryloxy ou un alcoxy inférieur, les restes alkyles inférieurs ou alcoxy inférieurs présentant 1 à 9 atomes de carbone et l'expression aryle prise en soi ou dans aryloxy représentant le phényle éventuellement substitué.

**6.** Procédé selon la revendication 5, caractérisé en ce que, dans le ligand de formule II, $R^9$ représente le groupe $-PO(R^{10})_2$.

**7.** Procédé selon la revendication 5 ou 6, caractérisé en ce que, dans le ligand de formule II, $R^8$ représente le phényle, le p-tolyle, le m-tolyle ou le 3,5-xylyle et $R^{10}$ représente le phényle, le p-tolyle, le m-tolyle, le p-alcoxy inférieur carbonylphényle ou le tert.butoxy.

**8.** Utilisation des complexes chiraux de rhodium et de diphosphines de formule I pouvant être obtenus selon les revendications 1 à 7, comme catalyseurs, dans les hydrogénations asymétriques.

**9.** Utilisation des complexes chiraux de rhodium et de diphosphines de formule I pouvant être obtenus selon les revendications 1 à 7 dans l'hydrogénation asymétrique de la cétopantolactone en pantolactone.